# EUROPEAN PATENT SPECIFICATION

(11) **EP 0 211 543 B1**
(45) Date of publication and mention of the grant of the patent: **14.09.1994**
(21) Application number: 86305459.9
(22) Date of filing: 16.07.1986
(51) Int. Cl.: C12N 1/21, C12N 15/31, A61K 39/108

(54) **Invasive microorganisms**
Invasive Mikroorganismen
Micro-organismes invasifs

(30) Priority: 31.07.1985 US 761222
(43) Date of publication of application: 25.02.1987
(73) Proprietor: THE BOARD OF TRUSTEES OF THE LELAND STANFORD JUNIOR UNIVERSITY, Palo Alto, California 94306 (US)
(72) Inventor: Isberg, Ralph R., Brookline Massachusetts 02146 (US); Falkow, Stanley, Portola Valley California 94025 (US)
(74) Representative: Harrison, David Christopher

(56) References cited:
- CANADIAN JOURNAL OF MICROBIOLOGY, vol. 21, no. 12, December 1975 National Research Council,Canada AKE BOVALLINS et al. "Ingestion and survival of Y.pseudotuborculosis in Hela cells" pages 1997-2007
- INFECTION AND IMMUNITY, vol. 37, no. 2, August 1982, American Society for Microbiology;Bethseda Md;BOLIN et al. "Temperature-Inducible Outer Membrane Protein of Yersinia pseudotuberculosis and Yersinia enterocolitica is Associated with the virulence Plasmid" pages 506-512
- INFECTION AND IMMUNITY, vol. 40, no. 1; April 1985, American Society for Microbiology;Bethseda Md; TH.L. HALE et al. "Characterization of Virulence Plasmids and Plasmid-Associated Outer Membrane Proteins in Shigella flexneri; Shigella sonnei, and Escherichia coli" pages 340-350
- INFECTION AND IMMUNITY, vol. 39, no. 3, March 1983, American Society for Microbiology;Bethseda Md; PH.J. SANSONETTI et al. "Alterations in th Pathogenicity of Escherichia coli K-12 After Transfer of Plasmid and Chromosomal Genes from Shigella flexneri" pages 1392-1402
- INFECTION AND IMMUNITY, vol. 49, no. 1, July 1985, American Society for Micorobiology, Bethseda Md; A.T. MAURELLI et al. "Cloning of Plasmid DNA Se-quences Involved in Invasion of HeLa Cells by Shigella flexneri" pages 164-171

## Description

For many species of bacteria, invasion and survival within mammalian cells is central to establishing a successful host-parasite relationship. This localization within host cells may protect the microorganism from host defenses and permit the microorganism to cross epithelial barriers and subsequently become systemically distributed. The precise mechanisms by which bacteria enter host tissues have been unclear. The invasive character of pathogens, while deleterious to the health and viability of host cells, does provide a mechanism for transfer of molecules and aggregates across an intact cellular membrane. Thus, if the invasive quality could be transferred to an innocuous microorganism strain, such a strain could serve as a vehicle for transporting molecules of interest into the cytoplasm and organelles of host cells.

There is a further consideration in that the bacterium could provide for the transfer of genetic material into the mammalian host cell. In this manner, novel genetic capabilities could be imparted to the host cell. One capability of interest would be the synthesis of surface membrane proteins or envelope proteins of pathogens. These proteins would then serve as antigens to provide a strong immune response, without the host having to suffer the effects of infection by the pathogen.

The invasion of epithelial cells by Yersinia pseudotuberculosis is reported by Bovallius and Nilsson, (1975) Can. J. Microbiol. 21:1997-2007 and Bolin et al., (1982) Infect. Immun. 37:506-512. The factors associated with Shigellae invasiveness are described by Hale et al. (1983) Infect. Immun. 40:340-350. Sansonetti et al., ibid (1983) 39:1392-1402 and Maurelli et al., ibid (1985) 49:164-171 describe the manipulation of the plasmid in Shigellae encoding functions essential for invasiveness.

### SUMMARY OF THE INVENTION

Methods and compositions are provided for introducing macromolecules into mammalian host cell using substantially non-pathogenic bacteria to which have been imparted novel invasive capability. The genetic capability of invasiveness is transferred from one bacterial host to another, whereby the recipient may now invade mammalian cells and be used as a carrier for various molecules. In this manner, molecules which cannot normally cross the cell membrane may be transported across the membrane while retaining an intact membrane and a viable host cell. In one application, the modified bacterium may be used as a vaccine by providing genetic capability for expressing surface membrane proteins or envelope proteins of various pathogens. A protocol is provided for screening invasive pathogens for the genetic sequences associated with such capabilities and transferring such genetic capabilities to non-pathogenic (innocuous) hosts.

### DESCRIPTION OF THE SPECIFIC EMBODIMENTS

The subject invention provides means for introducing exogenous molecules into mammalian cell hosts employing bacteria as the vehicle. The method involves isolating the genetic capability of invasiveness from an invasive bacterium and transferring the genetic capability to a recipient bacterium which does not naturally have the transferred gene imparting invasive capability, making any additional modifications in the modified recipient bacterium, as appropriate, and contacting mammalian cells susceptible to invasion by the modified recipient bacterium with the modified bacterium. A screening protocol is provided for identifying the genetic region associated with invasiveness, which region may be introduced directly into the recipient bacterium or may be further modified prior to introduction. The recipient bacterium may be used as a vehicle for introducing various molecules, particularly macromolecules, into a mammalian host susceptible to invasion.

The first aspect of the subject invention to be considered will be the selection protocol for identifying the genetic capability of invasiveness. An invasive bacterium is selected, the genome fragmented, either mechanically or by partial or complete digestion with one or more restriction enzymes, and the fragments joined to an appropriate replication system for introduction into a non-invasive bacterium. The replication system may be derived from a plasmid or a virus, desirably providing for a vector having a copy number equal to or greater than one and the copy number may be 200 or greater.

The vector should neither be lethal to the host nor result in an undue reduction in viability. Desirably, the vector should provide a marker which allows for selection of recipient bacteria which include the vector. Various markers are available, such as biocide resistance, e.g., antibiotic resistance and heavy metal resistance, genes imparting prototrophy to an auxotrophic host, immunity, and the like.

The genomic fragments inserted into the vector will usually be at least about 2kb and not more than about 50kb, generally ranging from about 5 to 20kb. Conveniently, a viral vector may be employed which provides for selection of fragments in the desired range based on the packaging requirements. While it is not essential to select for the bacteria which have received the vector, it is preferable to select for recipient bacteria. Desirably, the vectors should be capable of stable episomal maintenance or integration into the genome. Where integration is involved, amplification of the gene is desirable.

The genomes of invasive bacteria are mechanically sheared or digested with one or more restriction enzymes, either partially or completely, to provide fragments in the range of about 2 to 20kbp. The fragments are then inserted into an appropriate vector, such as a viral vector, e.g., cosmid, or plasmid vector, e.g., pBR322. Non-invasive bacteria are transfected or transformed with the vectors and modified organisms are selected by means of a marker, e.g., antibiotic resistance. The desired clones are then enriched by the following procedure.

The surviving bacteria are cloned, suspended in an appropriate nutrient medium and introduced onto confluent layers of invasive susceptible mammalian cells. The cells are allowed to incubate for a sufficient time, usually at least about 1 hour, and less than about 12 hours, conveniently from about 2 to 6 hours, under conditions which maintain viability of the cells. The monolayer is then stringently but carefully washed under conditions which remove nonadherent recipient bacteria, so that only adherent bacteria remain bound to the mammalian cell monolayer. The internalized cells are then released from the monolayer by treatment with a mild detergent, e.g., a nonionic detergent, generally at a concentration in the range of about 0.1 to 2%, more conveniently about 0.5 to 1.5%, in an aqueous medium. Any mild technique which allows for the viability of the bacterium with release of the bacterium cells from the mammalian cells may be employed. The released bacterial cells are then expanded and cloned.

Transposon mapping may be employed for identifying transposon insertions which destroy invasive capability. In this manner, the structural gene with its associated regulatory signals can be mapped to a particular site on the fragment. Other techniques involve employing partial digestions, cloning and screening for invasive capability, followed by sequencing and identifying specific sequences associated with transcription and translation as indicative of the structural gene and its associated regulatory signals.

If desired, the regulatory signals, particularly the transcription initiation signal, may be modified by the addition or substitution of the native transcriptional initiation region with a transcriptional initiation region associated with a different gene. In this way, one can provide for low or high levels of constitutive or inducible expression of the DNA sequence encoding for invasive capability. Various transcriptional initiation regions or promoters are available, which are temperature sensitive, are inducible in the presence of various metabolites or nutrients, and the like. Therefore, a transcriptional initiation region may be employed which is regulated by the bacterial host and the invasive capability may be activated or inactivated by physically or chemically changing the environment of the bacterial host. Thus, nutrients and metabolites such as glucose, tryptophan, histidine, galactose, lactose, may be employed to induce or repress the expression of the invasive gene (inv). The inducible transciptional regulatory region may be selected in accordance with the mammalian host, depending upon whether the coinducer or corepressor is naturally found in the mammalian host or can be administered to the host.

Constructs may then be prepared which may be used for introducing invasive capability into an appropriate bacterial host. Depending upon the purpose for invasiveness, a wide variety of bacterial hosts may be employed. The subject method provides for introduction of DNA capability into a mammalian cell where the bacterium is employed as the vehicle for introduction of the DNA capability into a mammalian cell. For example, a shuttle vector may be provided in the invasive bacterial host which has the capability for replication in the mammalian cell as well as the bacterium, where the shuttle vector may exist as an episomal element or become integrated into the mammalian cell genome. In this manner, bacterial hosts for cloning may be used directly for the transfer of DNA into a mammalian cell host with high efficiency. Thus, a wide variety of genetic capabilities can be introduced into mammalian hosts, for example, the expression of lymphokines, hormones, enzymes, surface membrane proteins, and the like, such as interferons, interleukins, growth factors, hydrolases, oxidoreductases, receptors, antibodies, histocompatability antigens, etc.

A second manner in which the invasive bacterium may be used is as a vaccine. For this purpose, in addition to the invasive genetic capability, genes encoding for surface membrane proteins, capsid proteins, or envelope proteins, singly or in combination may be introduced into the invasive modified bacterial host for injection into a mammal to induce an immune response. The genes encoding for the antigens may be obtained from a wide variety of pathogens, including viruses, prokaryotes, e.g., bacteria, eukaryotes, e.g., fungi, protists, or the like, or such intermediate species as chlamydia. In many cases, the gene of interest is known and available or may readily be isolated. Where not known, the techniques employed for identifying specific structural genes can be employed for identifying the genes coding for the desired antigen.

A third manner in which the invasive bacterium may be used is as a vehicle for the introduction of molecules, particularly macromolecules, into a mammalian cellular host, either in vitro or in vivo. For example, cytotoxic resistance provided by an enzyme could be transferred into cells or a cytotoxic agent, e.g., aminoglycosides, hybritoxins, etc., non-cytotoxic to the bacterium could be introduced into mammalian cells. Dyes or other contrast agents could be introduced into the cells for visualization of cell features. Labelled antibodies could be introduced into the cells to define the location of particular antigens. Other uses will also be apparent.

A large number of mammalian replication systems and vectors are available, particularly viral replication systems, such as SV-40, adenovirus, bovine papilloma virus, etc. See, for example, Southern and Berg, J. Mol. Appl. Genet. (1982) 1:327-341; and Mulligan and Berg, Proc. Natl. Acad. Sci. USA (1981) 78:2072-2076.

One technique would involve employing antisera from a mammalian host who had suffered from, particularly was undergoing infection by, the pathogen of interest. The pathogen in culture could be lysed and immunoprecipitated with the antisera from the mammalian host and electrophoresed. A cDNA or genomic library could be prepared from the pathogen. By at least partially sequencing the immunoprecipitated proteins, amino acid sequences could be identified, which could be translated into probes. The probes could then be used for screening the library and identifying sequences complementary to the probes. Once sequences which hybridize to the probes have been identified, where the regulatory sequences are recognized by a prokaryotic host, the prokaryotic host may be transformed with the sequence and the expression product identified. Where the expression of the structural gene is regulated by sequences which are not recognized by prokaryotic hosts, then some manipulation will be required in identifying the sequence coding for the particular antigen and inserting the sequence into an appropriate expression vector. A large number of expression vectors exist and various techniques are available for tailoring the structural gene to remove superfluous DNA sequences, particularly 5' to the structural gene. Techniques such as resection with Bal31, primer repair, and in vitro mutagenesis to introduce a convenient restriction site, have all been used successfully.

Antigens of interest may come from a wide variety of sources, such as bacteria, such as Bordatella, Salmonella, Neisseria, Pneumococcus, Shigellae, Yersinia, Cholera, Meningococcus, Listeria, Mycobacterium, etc,; viruses, such as HTLV-I, -II, and -III, FeLV, HSV-1 and -2, Adenovirus, Varicella, Vaccinia, Hepatitis, Influenza, Measles, Rubella, Smallpox, Typhoid, Yellow Fever, etc. fungi, such as Candida, Microsporum, Tricophyton, Arthroderma, Cryptococcus, Blastomyces, Histoplasma, Coccidroides, Paracoccidroides, Aspergillus, Phycomycetes, Sporotorax, Epidermophyton, etc. other pathogenic microorganisms, such as Chlamydia Giardia, etc.

The organisms may be administered in any convenient form as a vaccine. Normally, physiologically acceptable carriers will be employed, such as deionized water, phosphate buffered saline (PBS), aluminum hydroxide, sugar or the like. Usually, the dosage will be determined empirically; about 10⁴ to 10¹⁰ cells will be administered to a human host, with proportionate administration based on size to other mammalian hosts. Generally, there will be a first administration, followed by one or more administrations at two to six week intervals. The particular amount administered will depend upon a number of factors, such as the viability of the invasive microorganism in the host, the concentration of the antigen on the surface of the pathogen, the number of different antigens which are present, the level of immune response to the particular antigen(s), and the like. Administration may be orally, by injection, intravenously, intraarterially, subcutaneously, intraperitoneally, etc. The manner of administering live vaccines is well established and may be found in such texts as Basic and Clinical Immunology, eds. Stites, Stobo. Fudenberg and Wells. 4th ed. Lange Medical Publications, Los Altos, CA, 1982.

The gene coding for the invasive genetic capability may come from any convenient source. A significant number of organisms are known to be capable of invasion, such as Yersinia, Chlamydia, Legionella pneumophila, Listeria monocytogenes, Mycobacterium tuberculosis, Mycobacterium leprae, Salmonella typhosa, Brucella abortus, Cryptococcus neoformans, Histoplasma capsulation, Candida albicans, Tripanosoma cruze, Toxaplasma gondi, Leishmania donovani, etc. Thus the organisms may be bacterial. Preferred organisms will be those which provide a single gene which results in invasive capability. The affinity for the mammalian host receptor should be at least about 0.1. Of particular interest is Yersinia which is demonstrated in the Experimental section as paradigmatic of invasive bacteria. The gene providing the capability is referred to as inv with the phenotype INV.

The non-invasive bacterium which is modified to become invasive will be selected depending upon its ultimate purpose. Where the bacterium is to be the vehicle for transfer of DNA into mammalian cells in culture, then any convenient bacterium may be employed, particularly one which may be used for cloning of the DNA to be transferred. Therefore, many of the strains of E. coli, e.g., K12, maybe employed as the recipient bacterium which is modified to become invasive. Where the modified bacterium is to be employed as a vaccine, the host will normally be selected so as to be innocuous (non-pathogenic), to be capable of being viable extracellularly for an extended period of time, preferably at least about 3 days in the vaccinated host, and to be subject to ready clearance in the vaccinated host. Desirably, the modified recipient bacterium will be free of pyrogens, toxins, or other disease symptom causing factors. By innocuous is intended that regardless of the dose or route, no disease will be observed with an immunocompetent host. While pathogenic bacteria may be employed, particularly attenuated pathogenic bacteria, these are not preferred, since there is a possibility of reversion to pathogenicity. Bacterial hosts which may be used for modification to invasive capability include besides E. coli, members of the genus Staphylococci, Pneumococci, Streptococci, e.g., mutans, Neisseria, e.g., catarrhalis, Veillonella, Lactobacilli, Corynebacteria, Clostridia, Hemophilic bacilli, Bacteroides, Actinomycetes, Spirochetes, Mycoplasma, etc.

The manner in which the genetic capability for invasiveness is introduced into the recipient bacterium may be any of the convenient techniques including transformation, e.g., calcium precipitated DNA, transfection, transduction, conjugation, fusion, etc. Applicable techniques may be found in Maniatis et al., A Laboratory Manual, Cold Spring Harbor Laboratory, Cold Spring Harbor, New York, 1982. As already indicated, a marker will normally be present which allows for selection of those bacteria which have received the invasive genetic capability. The bacteria may then be grown in an appropriate nutrient medium and used as appropriate.

The invasive bacteria may be used to prepare antisera for passive immunization. Thus, γ-globulin could be prepared which has antibodies to a broad spectrum of pathogens and for strains of a particular pathogen. The γ-globulin may be isolated and purified from serum by ammonium sulfate precipitation and fractionation according to known techniques. Administration to a mammalian host will generally be in amounts of 50 to 500 mg/kg of host in any physiologically acceptable carrier. Administration will usually be by injection, e.g., intraveneously. The modified recipient bacterium may also be used in assays for detecting the presence of antibodies to the antigens foreign to the modified bacterium or the antigens themselves. They also may find use in competing with the invasive bacteria so as to be useful for therapy.

In addition, the subject bacteria may be used for expression in proteins expressed by the inv gene. Particularly, by having high copy number vectors, the modified foreign bacteria may be harvested, lysed and the inv antigen isolated by conventional ways, e.g., affinity chromatography, electrophoreses, chromatography, etc.

### EXPERIMENTAL

The following examples are offered by way of illustration and not by way of limitation.

### Enrichment for Invasive Phenotypes

A cosmid bank was constructed (Hohn, Methods in Enzymology (1979) 68:299-309; Koomey et al. Proc. Natl. Acad. Sci. USA (1982) 79:7881-7885) by ligating a size-fractionated Sau3AI digest of chromosomal DNA isolated from Y. pseudotuberculosis strain YPIII (p⁻) (Bolin and Wolf-Watz, Infect. Immun. (1984) 43:72-78) into the BamHI site of pREG153 (available from Dr. R. Gill) a cosmid cloning vehicle that encodes resistance to ampicillin and is further characterized by its low copy number (5-8). Aliquots of the ligation mixture were packaged in vitro (Enquist and Sternberg, Methods in Enzymology (1979) 68:281-298) into phage lambda heads with an extract prepared from MMSA345 (available from Dr. F. Stahl) and then infected into E. coli K12 strain HB101 (Maniatis et al., A Laboratory Manual), selecting for ampicillin-resistant transductants. Pooled bacteria were grown overnight in culture, washed twice in sterile PBS and resuspended to a density of 5X10⁸ bacteria ml⁻¹. The washed bacteria (2ml) were then introduced onto a confluent monolayer containing 1X10⁷ HEp-2 cells grown in RPMI1640 medium (Irvine Biologicals), and incubated for 3 hours at 36°C in a 5% CO₂ atmosphere. Monolayers were then washed 10 times with sterile PBS to remove non-adherent bacteria. Bacterial clones remaining associated with the monolayer were released with 1% Triton X100® in deionized water, and plated for individual colonies on bacteriological medium.

### Screen for Invasive Cultures

Saturated cultures of the transformed bacteria were grown at 28°C, washed twice in PBS and resuspended to a concentration of 3X10⁸ bacteria ml⁻¹. Aliquots (50 µl) of each strain were added to monolayer cultures of HEp-2 cells seeded at a concentration of 2X10⁵ animal cells per microtiter well (24 well, Falcon 3047 microtiter dishes) in RPMI1640 medium. Bacteria were centrifuged onto the monolayer at 600Xg (Devenish and Schiemann, Infect. Immun. (1981) 32:48-55), and the infected cultures were incubated at 36°C for 3 hours in 5% CO₂ atmosphere, to allow binding and invasion of bacteria. Non-adherent bacteria were removed from the monolayer by washing three times with sterile PBS, and RPMI medium containing 40 µg ml⁻¹ gentamicin (Sigma Chemical) was added to each microtiter well. The incubation was continued at 36°C for 2 hours in the presence of the antibiotic, before washing the monolayers twice more with PBS. Internalized bacteria were then released from the monolayers by the addition of 1% Triton X100® and titered on L agar plates (Miller, Experiments in Molecular Genetics, Cold Spring Harbor Laboratory, Cold Spring Harbor, New York, (1972)).

The following Table indicates the results.

**TABLE 1**

| Enrichment procedure yields E. coli strains that invade cell culture monolayers | |
|---|---|
| Strain | % Invasion^{d} |
| YPIII(p⁻)^{a} | 9.0 |
| HB101^{b} | 0.005 |
| HB101(pINVA2)^{c} | 8.3 |
| HB101(pINVA7) | 7.9 |
| HB101(pINVG10) | 8.7 |
| HB101(pRI203) | 9.2 |

| | |
|---|---|
| ^{a} Yersinia pseudotuberculosis strain (Bolin et al. supra.), | |
| ^{b} E. coli K12 strain HB101. | |
| ^{c} HB101 harboring cosmids that are denoted in parenthesis). | |
| ^{d} Percentage of bacteria added to HEp-2 monolayers that resist treatment by gentamicin. | |

It was found that 12 of the 22 candidate strains that survived the enrichment were invasive, based on the above. It is noteworthy that the efficiency of escape from gentamicin treatment, which may be equated with bacterial invasion, was similar to that found for the Y. pseudotuberculosis strain used as the DNA donor.

To determine if isolated bacterial derivatives could invade cultured animal cells, ultrathin sections of monolayer cells exposed to several of the bacterial strains were analyzed by electron microscopy (Horwitz, J. Exp. Med. (1983) 158:1319-1331). Tissue culture cells (Falcon 3046, 6 well dishes) seeded with 8X10⁵ HEp-2 cells in RPMI 1640 were incubated in the presence of 6X10⁻⁷ bacteria for 3 hours at 36°C. Monolayers were then washed 10 times with PBS and incubated for 10 minutes at 37°C in the presence of PBS containing 0.1mM EDTA. The monolayers were gently washed once more with PBS in the presence of EDTA, suspended in 1ml of PBS, and pelleted at 600Xg for 10 minutes. The cell pellets were successively fixed with 2% glutaraldehyde and 2% osmium tetroxide in 0.1M cacodylate buffer (pH 7.4), before staining with uranyl acetate. Samples dehydrated in ethanol were embedded in Spurrs (Polysciences), thin sectioned, stained successively with 1% uranyl acetate and lead acetate (Reynolds, J. Cell. Biol. (1963) 17:208-213), and visualized with a Phillips 201c electron microscope. An E. coli K12 HB101 strain is unable to enter HEp-2 cells. In contrast, the same bacterial strain harboring an intact inv locus showed a large number of bacteria associated with the animal cell. These bacteria appeared to be both bound to the outside of the cell as well as present within large endocytic vesicles. Furthermore, when E. coli HB101 cells were employed which harbor the pRI203 plasmid with an intact inv locus, invasion was observed, whereas the same bacteria which harbored the plasmid pRI203.14::Tn5, which has a Tn5 insertion mutation in the inv locus were compared, no invasion was observed. Therefore, invasiveness is only observed with a functionally intact inv locus. The number of intracellular bacteria had substantially diminished and the invaded cells remained viable.

The locus of the inv gene was established as follows. One plasmid, pINVA2 was analyzed by causing a series of Tn5 insertion mutations in the cosmid DNA, taking the precaution that each mutation analyzed was the result of an independent event. Mutations were induced by the kanamycin-resistance transposon Tn5 via transposition from λb221rex::Tn5 cI857Oam23Pam80 onto pINVA2 according to the method of deBruijn and Lupski, Gene (1984) 27:313-149. To select for insertions onto the cosmid, kanamycin-resistant colonies were pooled together and infected lytically with λlac5 imm21cts. The resulting lysate was used to transduce HB101 to simultaneous kanamycin- and ampicillin-resistance. Such transductants contained insertions of Tn5 at random sites on the cosmid. Several hundred such transductants were assayed for invasiveness as described previously, and the map position of 20 mutants that eliminated invasiveness and 20 insertions that had no effect on the invasive phenotype were determined. In order to ensure that each insertion was independent, the physical location of no more than one mutation from each pool was analyzed. The sensitivity to insertion inactivation of the invasive phenotype was mapped to a contiguous 3.2kb region, which region was shown to code for a single large protein which was indicated as being 108kdal by electrophoresis using myosin, β-galactosidase and phospholipase G as standards.

The above data demonstrate that the invasive (inv) genetic capability can be transferred from one host to another, so that the phenotype may be imparted to a bacterial host which lacks the particular phenotype. Furthermore, the mammalian cells are able to endocytose the entire cell based on the presence of a particular structure encoded by inv on the surface membrane. In this manner, non-invasive bacteria can be modified to become invasive bacteria, which capability can be used for diverse purposes, such as the introduction of exogenous DNA or other molecules into mammalian hosts, induction of an immune response to one or a plurality of antigens associated with pathogens, so as to be useful as vaccines, for production of antisera having a spectrum of antibodies to a spectrum of pathogens, and for the production of proteins which may be used to inhibit invasion of pathogens in mammalian host cells.

Although the foregoing invention has been described in some detail by way of illustration and example for purposes of clarity of understanding, it will be obvious that certain changes and modifications may be practiced.

## Claims (Claims for the following Contracting State(s): BE, CH, DE, FR, GB, IT, LI, LU, NL, SE)

1. A bacterium capable of invading mammalian cells having invasive phenotype attributable to a single membrane protein, the said protein being expressed as a result of the introduction of exogenous DNA into said bacterium or progenitor thereof.

2. A bacterium according to Claim 1, wherein said bacterium has at least one additional exogenous gene expressing a surface membrane protein, a capsid protein or envelope protein of at least one pathogen capable of infecting an invasive susceptible mammalian host.

3. A bacterium according to Claim 1, wherein said bacterium is E. coli.

4. A bacterium according to Claim 1, wherein said bacterium contains a plasmid having a mammalian replication system.

5. A bacterium according to Claim 4, wherein said plasmid includes a gene capable of expressing a mammalian protein.

6. A bacterium according to Claim 1, containing a chemical not naturally found in said bacterium other than said membrane protein.

7. A vaccine comprising a bacterium according to Claim 2, in an amount sufficient to provide an immune response from an immunocompetent susceptible mammalian host and a physiologically-acceptable carrier.

8. A vaccine according to Claim 7, wherein said bacterium is E. coli.

9. An E. coli strain containing the gene coding for the invasiveness (inv)-phenotype from Yersinia.

10. A method for producing the expression product of the Yersinia inv gene, which comprises:
growing in a nutrient medium an E. coli strain containing a copy of the Yersinia inv gene capable of expression whereby said expression product is produced;
harvesting said E. coli;
lysing said harvested E. coli and isolating said expression product.

11. Antisera produced in response to immunization with a vaccine according to Claim 6.

## Claims (Claims for the following Contracting State(s): AT)

1. Method of producing a bacterium capable of invading mammalian cells having invasive phenotype attributable to a single membrane protein by the introduction of exogenous DNA into said bacterium or progenitor thereof resulting in the expression of the said protein.

2. A method according to Claim 1, wherein said bacterium has at least one additional exogenous gene expressing a surface membrane protein, a capsid protein or envelope protein of at least one pathogen capable of infecting an invasive susceptible mammalian host.

3. A method according to Claim 1, wherein said bacterium is E. coli.

4. A method according to Claim 1, wherein said bacterium contains a plasmid having a mammalian replication system.

5. A method according to Claim 4, wherein said plasmid includes a gene capable of expressing a mammalian protein.

6. A method according to Claim 1, wherein the bacterium contains a chemical not naturally found in said microorganism other than said membrane protein.

7. A method of preparing a vaccine comprising using a bacterium obtained by the method of Claim 2 or Claim 3, in an amount sufficient to provide an immune response from an immunocompetent susceptible mammalian host and a physiologically-acceptable carrier.

8. A method for producing the expression product of the Yersinia inv gene, which comprises:
growing in a nutrient medium an E. coli strain containing a copy of the Yersinia inv gene capable of expression whereby said expression product is produced;
harvesting said E. coli;
lysing said harvested E. coli and isolating said expression product.

## Patentansprüche (Patentansprüche für folgende(n) Vertragsstaat(en): BE, CH, DE, FR, GB, IT, LI, LU, NL, SE)

1. Bakterium, das fähig ist, in Säugetierzellen einzudringen und einen invasiven Phänotyp aufweist, der einem einzelnen Membranprotein zugeschrieben werden kann, wobei das genannte Protein als Folge der Einführung exogener DNA in das genannte Bakterium oder seinen Vorgänger exprimiert wird.

2. Bakterium nach Anspruch 1, worin das genannte Bakterium zumindest ein zusätzliches exogenes Gen aufweist, das ein Oberflächenmembranprotein, ein Capsidprotein oder Hüllenprotein von zumindest einem Pathogen exprimiert, das einen invasionsanfälligen Säugetierwirt infizieren kann.

3. Bakterium nach Anspruch 1, worin das genannte Bakterium E.coli ist.

4. Bakterium nach Anspruch 1, worin das genannte Bakterium ein Plasmid enthält, das ein Säugetierreplikationssystem aufweist.

5. Bakterium nach Anspruch 4, worin das genannte Plasmid ein Gen enthält, das ein Säugetierprotein exprimieren kann.

6. Bakterium nach Anspruch 1, das einen anderen chemischen Stoff als das genannte Metallprotein enthält, der im genannten Bakterium nicht natürlich vorkommt.

7. Impfstoff enthaltend ein Bakterium nach Anspruch 2 in einer Menge, die ausreicht, um für eine Immunreaktion von einem immunkompetenten anfälligen Säugetierwirt zu sorgen, sowie einen physiologisch verträglichen Träger.

8. Impfstoff nach Anspruch 7, worin das genannte Bakterium E.coli ist.

9. E.coli Stamm, der das Gen enthält, das für den Invasivitäts- (inv) Phänotyp von Yersinia kodiert.

10. Verfahren zum Herstellen des Expressionsprodukts des Yersinia inv-Gens, das umfaßt:
das Züchten in einem Nährmedium eines E.coli-Stamms, der eine Kopie des Yersinia inv-Gens enthält, das exprimierbar ist, wodurch das genannte Expressionsprodukt hergestellt wird;
das Ernten der genannten E.coli;
das Lysieren der geernteten E.coli und das Isolieren des genannten Expressionsprodukts.

11. Antiseren, die als Reaktion auf die Immunisierung mit einem Impfstoff nach Anspruch 6 erzeugt werden.

## Patentansprüche (Patentansprüche für folgende(n) Vertragsstaat(en): AT)

1. Verfahren zum Herstellen eines Bakteriums, das in Säugetierzellen eindringen kann und einen invasiven Phänotyp aufweist, der einem einzelnen Membranprotein zugeschrieben werden kann, durch Einführung exogener DNA in das genannte Bakterium oder seinen Vorgänger, was zur Expression des genannten Proteins führt.

2. Verfahren nach Anspruch 1, worin das genannte Bakterium zumindest ein zusätzliches exogenes Gen aufweist, das ein Oberflächenmembranprotein, ein Capsidprotein oder Hüllenprotein von zumindest einem Pathogen exprimiert, das einen invasionsanfälligen Säugetierwirt infizieren kann.

3. Verfahren nach Anspruch 1, worin das genannte Bakterium E.coli ist.

4. Verfahren nach Anspruch 1, worin das genannte Bakterium ein Plasmid enthält, das ein Säugetierreplikationssystem aufweist.

5. Verfahren nach Anspruch 4, worin das genannte Plasmid ein Gen enthält, das ein Säugetierprotein exprimieren kann.

6. Verfahren nach Anspruch 1, worin das Bakterium einen anderen chemischen Stoff als das genannte Membranprotein enthält, der im genannten Bakterium nicht natürlich vorkommt.

7. Verfahren zur Herstellung eines Impfstoffs, umfassend die Anwendung eines nach dem Verfahren nach Anspruch 2 oder 3 hergestellten Bakteriums in einer Menge, die ausreicht, um für eine Immunreaktion von einem immunkompetenten anfälligen Säugetierwirt zu sorgen, sowie eines physiologisch verträglichen Trägers.

8. Verfahren zum Herstellen des Expressionsprodukts des Yersina inv-Gens, das umfaßt:
das Züchten in einem Nährmedium eines E.coli-Stamms, der eine Kopie des Yersinia inv-Gens enthält, das exprimierbar ist, wodurch das genannte Expressionsprodukt hergestellt wird;
das Ernten der genannten E.coli;
das Lysieren der geernteten E.coli und das Isolieren des genannten Expressionsprodukts.

## Revendications (Revendications pour l'(les) Etat(s) contractant(s) suivant(s): BE, CH, DE, FR, GB, IT, LI, LU, NL, SE)

1. Bactérie capable d'envahir des cellules mamaliennes ayant un phénotype d'invasion attribuable à une protéine membrane unique, ladite protéine étant exprimée comme un résultat de l'introduction d'un ADN exogène dans ladite bactérie ou la souche de celle-ci.

2. Bactérie selon la revendication 1, dans laquelle ladite bactérie a au moins un gène exogène additionnel exprimant une protéine de membrane de surface, une protéine capside ou une protéine enveloppe d'au moins un pathogène capable d'infecter un hôte mamalien susceptible d'invasion.

3. Bactérie selon la revendication 1, dans laquelle ladite bactérie est E. coli.

4. Bactérie selon la revendication 1, dans laquelle ladite bactérie contient un plasmide ayant un système de réplication mamalien.

5. Bactérie selon la revendication 4, dans laquelle ledit plasmide inclut un gène capable d'exprimer une protéine mammalienne.

6. Bactérie selon la revendication 1, contenant un produit chimique ne se trouvant pas naturellement dans ladite bactérie autre que ladite protéine membrane.

7. Vaccin comprenant une bactérie selon la revendication 2, en une quantité suffisante pour fournir une réponse immune d'un hôte mammalien susceptible d'immunocompétence et un porteur acceptable physiologiquement.

8. Vaccin selon la revendication 7, dans lequel ladite bactérie est E. coli.

9. Souche de E. coli contenant la gène codant pour le phénotype d'invasivité (inv) provenant de Yersinia.

10. Méthode pour produire le produit d'expression du gène inv Yersinia, qui comprend :
la croissance dans un milieu de nutriment d'une souche de E. coli contenant une copie du gène inv Yersinia capable d'expression ce par quoi ledit produit d'expression est produit;
la récolte dudit E. coli;
la lyse dudit E. coli moissonné et l'isolement dudit produit d'expression.

11. Antisérum produit en réponse à l'immunisation avec un vaccin selon la revendication 6.

## Revendications (Revendications pour l'(les) Etat(s) contractant(s) suivant(s): AT)

1. Méthode de production d'une bactérie capable d'envahir des cellules mammaliennes ayant un phénotye invasif attribuable à une protéine membrane unique par l'introduction d'un ADN exogène dans ladite bactérie ou la souche de celle-ci résultant en l'expression de ladite protéine.

2. Méthode selon la revendication 1, dans laquelle ladite bactérie a au moins un gène exogène additionnel exprimant une protéine membrane de surface, une protéine capside ou une protéine enveloppe d'au moins un pathogène capable d'infecter un hôte mammalien susceptible d'invasion.

3. Méthode selon la revendication 1, dans laquelle ladite bactérie est E. coli.

4. Méthode selon la revendication 1, dans laquelle ladite bactérie contient un plasmide ayant un système de réplication mammalien.

5. Méthode selon la revendication 4, dans laquelle ledit plasmide inclut un gène capable d'exprimer une protéine mammalienne.

6. Méthode selon la revendication 1, dans laquelle la bactérie contient un produit chimique non trouvé naturellement dans ledit microorganisme autre que ladite protéine membrane.

7. Méthode de préparation d'un vaccin comprenant l'utilisation d'une bactérie obtenue par la méthode de la revendication 2 ou 3, en une quantité suffisante pour fournir une réponse immune d'un hôte mammalien susceptible d'immunocompétence et un porteur acceptable physiologiquement.

8. Méthode pour produire le produit d'expression du gène inv Yersinia, qui comprend :
la croissance dans un milieu de nutriment d'une souche de E. coli contenant une copie du gène inv Yersinia capable d'expression ce par quoi ledit produit d'expression est produit;
la récolte dudit E. coli;
la lyse dudit E. coli moissonné et l'isolement dudit produit d'expression.
